# EUROPEAN PATENT APPLICATION

(11) **EP 1 619 253 A1**
(43) Date of publication of application: **25.01.2006**
(21) Application number: 04716354.8
(22) Date of filing: 02.03.2004
(51) Int. Cl.: C12N 15/85, C12N 5/08, A01K 67/02

(54) **GENE TRAP SYSTEM**

(30) Priority: 13.03.2003 JP 2003068307
(71) Applicant: Japan Science and Technology Agency, Kawaguchi-shi, Saitama 332-0012 (JP)
(72) Inventor: SUZUKI, Noboru, Tsu-shi, Mie 514-0063 (JP)
(74) Representative: Wilson Gunn
(86) International application number: PCT/JP2004/002569
(87) International publication number: WO 2004/081218

(57) **Abstract**

The present invention provides a gene-trap system, which can trap any gene including transiently expressed genes. A gene can be trapped by a trap vector, which employs Cre recombinase from bacteriophage P1, and the expression of the trapped gene is converted to constitutive expression of another reporter gene.

SEQUENCE LISTING

<110> Japan Science and Technology Agency

<120> Gene trap system

<130> PS04-405PCT

<160> 11

<170> PatentIn version 3.3

<210> 1
<211> 58
<212> DNA
<213> murine

<400> 1
agatctcact tagcccatcc atttggaatc tcacctttct tcctgtttct ttctgcag 58

<210> 2
<211> 31
<212> DNA
<213> artificial sequence

<220>
<223> primer

<400> 2
aactgcagcg ctccggttat tttccaccat a 31

<210> 3
<211> 32
<212> DNA
<213> artificial sequence

<220>
<223> primer

<400> 3
ccctcgagta ttatcatcgt gtttttcaaa gg 32

<210> 4
<211> 55
<212> DNA
<213> artificial sequence

<220>
<223> primer

<400> 4
catcatgagc ggccctccaa aaaagaagag aaaggtagaa gacccgggcg gccgc 55

<210> 5
<211> 129
<212> DNA
<213> artificial sequence

<220>
<223> splicing donor

<400> 5
tctagaattc gctgtctgcg agggccggct gttggggtga gtactccctc tcaaaagcgg 60

gcatgacttc tgcgctaaga ttgtcagttt ccaaaaacga ggaggatttg atattcacct 120

ggcccgcgg 129

<210> 6
<211> 10
<212> DNA
<213> artificial sequence

<220>
<223> primer

<400> 6
ggtttaaacc 10

<210> 7
<211> 35
<212> DNA
<213> artificial sequence

<220>
<223> primer

<400> 7
gactcgagtc gacatcgatt tttttttttt ttttt 35

<210> 8
<211> 19
<212> DNA
<213> artificial sequence

<220>
<223> primer

<400> 8
gactcgagtc gacatcgat 19

<210> 9
<211> 20
<212> DNA
<213> artificial sequence

<220>
<223> primer

<400> 9
gcgttggcta cccgtgatat 20

<210> 10
<211> 18
<212> DNA
<213> artificial sequence

<220>
<223> primer

<400> 10
gactcgagtc gacatcga 18

<210> 11
<211> 21
<212> DNA
<213> artificial sequence

<220>
<223> primer

<400> 11
ccttcttgac gagttcttct g 21

## Description

### Field of the Invention

The present invention relates to a method for searching a gene, which is expressed under a certain condition, and more specifically, relates to a gene-trap system, which immobilizes a transient expression of a gene under a certain condition as a constitutive expression of a reporter gene.

### Prior Art

Most of chromosomal DNA sequence has been made clear by the human genome project. However, it is required to comprehensively search functional genes related directly to physiological function, to develop new aspects in the fields of drug discovery and safety test.

As methods for comprehensively trapping transcribed and functional genes inside a specific cell type (expressed genes), three methods such as enhancer, promoter- and exon-trap have been reported. However, since the trap vector in the previous methods itself accompanies a reporter function (an indicator function of a gene-trap), it is possible to trap genes constitutively and stably expressed, but not to trap genes transiently expressed in the process of cellular changes such as differentiation and malignant transformation (Nature 1998 Apr 9; 392 (6676): 608-11).

### Problems to be solved by the Invention

The purpose of the present invention is to provide a gene-trap system (a method for gene trap), which can trap any gene including transiently expressed genes in the process of cellular changes such as differentiation and malignant transformation.

### Means to solve the Problems

The method of the present invention includes trapping genes by a trap vector, which employs Cre recombinase from bacteriophage P1, and converting the expression of the trapped gene to the constitutive expression of an added reporter gene. As the result, the method made it possible to search comprehensively "transiently expressed genes in the process of cellular physiological changes", which were previously impossible to be trapped, as well as genes previously trapped. The system makes it possible to construct a database understanding dynamic changes of gene expression.

The present invention is a method for gene-trap comprising steps of (the first step:) transforming target cells with a reporter vector, (the second step:) transfecting the transformed cells in the previous step with a trap vector, (the third step:) selecting cells without reporter activity from cells obtained in the previous step, (the fourth step:) exposing the selected cells in the previous step to a given condition and (the fifth step:) selecting the cells with reporter activity from the cells of the previous step, wherein the reporter vector having a unit composed by connecting, in sequence, promoters functional in the target cells, the first LoxP sequence, drug resistance gene, STOP sequence for transcription termination, the second LoxP sequence and a reporter gene, and the gene-trap vector having a unit composed by connecting, in sequence, splicing acceptor (SA), internal ribosomal entry site (IRES), Cre added with nuclear localization signal (nl-Cre), the first splicing donor (SD), constitutively expressing gene promoter, drug resistance gene and the second splicing donor (SD).

Furthermore, the method may further comprise the sixth step of cloning the DNA proximal to the trap vector in the cells selected in the fifth step and determining the nucleotide sequence.

Moreover, the present invention is a set of vectors for gene-trap comprising a reporter vector and a gene-trap vector, wherein the reporter vector comprises a unit composed by connecting, in sequence, promoters functional in the target cells, the first LoxP sequence, drug resistance gene, STOP sequence for transcription termination, the second LoxP sequence and a reporter gene, and the gene-trap vector comprises a unit composed by connecting, in sequence, splicing acceptor (SA), internal ribosomal entry site (IRES), Cre added with nuclear localization signal (nl-Cre), the first splicing donor (SD), constitutively expressing gene promoter, drug resistance gene and the second splicing donor (SD).

Still furthermore, the present invention is the cells transformed by the set of vector or non-human animal containing the cells.

### Brief Description of the Drawings

Figure 1 shows the basic unit of the trap vector of the present invention.
Figure 2 shows the basic unit of the reporter vector of the present invention.
Figure 3 shows the basic unit of the reporter vector constructed in Example 2.
Figure 4 shows a schematic diagram explaining each step in Example 4. Marks A and B represent LacZ positive samples and mark B shows that marked cells contain a trapped gene, which induce the expression of Cre gene in the trap vector during differentiation of ES cells.
Figure 5 represents the photograph of the cells, which shows LacZ positive only after induction of differentiation. LacZ positive cell mass is detected at the center of cell clump (arrowhead), and the color is dark blue. These cells correspond to mark B of Figure 4-right.

### Detailed Description of the Invention

The system of the present invention comprises two elements: trap vector and reporter vector.

### (1) Trap vector: pMIE

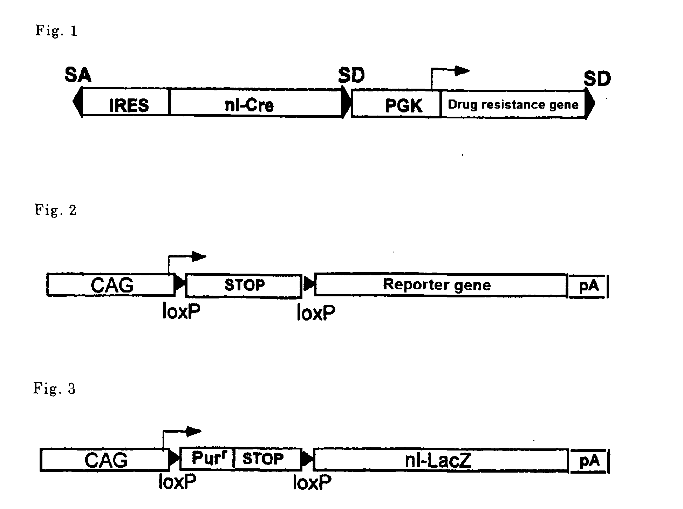

The basic unit of this trap vector is shown in Fig. 1. The basic unit structurally composed of roughly two parts.
The first half part is composed by connecting, in sequence, splicing acceptor (SA), internal ribosomal entry site (IRES), Cre added with nuclear localization signal (nl-Cre), and the first splicing donor (SD).
The second half part is composed by connecting, in sequence, constitutively expressing gene promoter (e.g. PGK gene promoter), drug resistance gene (e.g. Neo gene) and the second splicing donor (SD)
Furthermore, it is possible to insert functionally inactive DNA sequence between each element in these parts.
Splicing acceptor (SA) is a signal sequence located downstream of an intron necessary for splicing (a step to cut out an intron and to ligate the part coding an amino acids) as a posttranscriptional control.
Splicing donor (SD) is "a splicing controlling cis-element" as SA and is a functional sequence located upstream of intron.
IRES is the complex forming or entry site of ribosome to initiate protein synthesis. IRES is a part of human encephalomyocariditis virus genome and isolated PCR products from corresponding part of a commercial vector can be used as IRES.
Cre is a recombinase.
PGK gene promoter is a promoter of a housekeeping gene, which is not easily affected by chromosomal embedded site (the site trapped).
Drug resistance gene is any gene, whose coding protein counteracts drug toxic effects to mammalian cells. Neo resistance gene is most generally used.
These units are introduced to an appropriate vector. As a vector, Bluescript SK II (Stratagene) could be used.
A trap vector has the following functional characteristics.
The splicing acceptor in the first half enables 5' (upstream side) exon trap in addition to promoter trap of genes.
Genes without translation initiation sequence captured by IRES are guaranteed their translation from Cre gene, which does not depend on translation initiation based on cap structure of mRNA.

Drug resistance gene in the second half accompanies splicing donor downstream but not poly A addition signal. Therefore, selection based on a drug resistance enables trap of 3' exon and poly A addition signal. RNA isolated from trapped ES cell clone enables amplification and rapid identification of a part of trapped gene by 3' RACE using the drug resistance gene sequence as a primer.
A trap vector without poly A addition signal enables the screening with low background. Furthermore, RNA isolated for trapped EC cell clone enables amplification and rapid identification of a part of the nucleotide sequence of trapped gene by 3' RACE.

### (2) Reporter vector: pRMIE

As shown in Fig. 2, the reporter vector comprises a unit composed by connecting, in sequence, promoters functional in the target cells, the first LoxP sequence, drug resistance gene, STOP sequence for transcription termination, the second LoxP sequence and a reporter gene. Furthermore, it is possible to insert non-functional DNA sequence between each element in the unit.
STOP sequence for transcription termination could be either genes containing stop codon (cDNA) or poly A addition signal. A part of commercial vector, containing poly A addition signal and translation stop codon, could be used.
These units are introduced to an appropriate vector. As a vector, Bluescript SK II (Stratagene) could be used.

### The method of the present invention comprises the following steps:

The first step: In this step, such target cells as changeable between two cellular states like differentiation or malignant transformation are transfected with the reporter vector and cell lines are established. Any cells could be used as target cells, but embryonic stem cells (ES cells) have the following triple merits.
It is possible to search transiently expressed key genes for cell differentiation during divergent cell differentiation of pluripotent ES cells.
Since a trapped gene usually leads to functional abnormality (deletion or reduction), it is possible to construct mutated animals using trapped ES cell clones.
Since Cre gene is substituted with the expression of a trapped gene based on the structure of the trap vector of the present invention, animals constructed by trapped ES cell clones could be utilized as "Cre animals" (animals necessary for tissue- and cell-type specific genetic recombination).

The second step: In this step, the trap vector is transduced to target cells transformed in the previous step.

The third step: Cells without reporter activity are selected from cells obtained by the second step.

The fourth step: Exposing the selected cells in the previous step to a given condition, it is possible to particularly examine transiently expressed genes in the cells in response to the new condition.

The fifth step: The cells with reporter activity are selected from the cells of the previous step. In this step, it is sufficient to assay the activity by an appropriate method depending on the nature of the reporter gene.

The sixth step: DNA proximal to the trap vector in the cells selected in the previous step is cloned and the sequence is determined. This step can be done by a conventional method. Additionally, only to obtain the cells selected in the fifth step may skip this step.

The following Examples illustrate this invention, however, these are not constructed to limit the scope of this invention.

### Example 1: Construction of a trap vector.

A trap vector (pMIE-nlCre, Fig. 1) was constructed by connecting, in sequence, splicing acceptor (SA), internal ribosomal entry site (IRES), Cre with nuclear localization signal (nl-Cre), splicing donor (SD), PGK gene promoter, drug resistance gene (e.g. Neo) and splicing donor (SD) between XbaI-SalI sites in BluescriptSKII(-) (a cloning vector, Stratagene, USA).

As a splicing acceptor, the acceptor region of the second exon in type II ryanodine acceptor gene (SEQ ID: NO1, about 50 bp DNA of BgIII-PstI fragment, isolated and purified from commercial genomic library "LamdaFIXII" from Stratagene, USA) was used.

As IRES, a PCR fragment amplified using primers, shown by SEQ ID: NO 2 and 3, from corresponding part of commercial pCITE expression vector (Novagen) was used.

Since recombinant Cre protein is functional in nuclei, nl-Cre with nuclear localization signal (SEQ ID: NO 4) of SV40 T antigen upstream of the sequence coding a translation initiation site was used as Cre gene.

As PGK promoter, a promoter of mouse phosphoglycerokinase gene, which is not influential to transcriptional control of integrated gene, was used.

PCR amplified XbaI-SacII fragment (SEQ ID NO: 5) of synthetic intron in pOG44 (O'Gorman S, Fox DT, Wahl GM(1991), Recombinase-mediated gene activation and site-specific integration in mammalian cells. Science, 251(4999) 1351-1355) was used as a splicing donor.

Using synthetic oligomer (SEQ ID NO: 6), SpeI site of BluescriptSKII (-) was changed to PmeI site to separate BluescriptSKII (-) portion from the trap vector before introducing into target cells.

### Example 2: Construction of a reporter vector (pRMIE-nlLacZ)

The reporter vector pRMIE-nlLacZ was prepared by inserting and connecting, in sequence, CAG promoter functional in all types of target cells, the first LoxP sequence, puromycin resistance gene (Pac), STOP sequence for transcription termination, the second LoxP sequence, E. coli beta-galactosidase gene and polyA addition signal between NotI-Sall sites of the vector BluescriptSKII(-) for gene cloning (Fig. 3).

CAG promoter is a synthetic promoter comprising an enhancer of cytomegaro virus, chicken beta-actin promoter and an exon of rabbit beta-globin gene. STOP sequence is HindIII-BamHI fragment (about 1.3 Kb) derived from pBS302 (GENEBANK NO: U51223).

E. coli beta-galactosidase gene was added nuclear localization signal.

Due to this structure, the presence of Cre protein leads to recombination of LoxP sequence, cutout of STOP sequence and the transcription of beta-galactosidase gene located downstream starts.

### Example 3: Transduction of a reporter vector into ES cells and Selection of reporter-cells.

RW4 (Genome System) was used as ES cell. Procedure of cell culture and targeting was essentially according to the method reported (Suzuki N et. al., Development., 122 (11), 3587-95 (1996)).

Firstly, 5×10⁷ ES cells and 50 µg of reporter vector, after digestion with NotI and Sall, were mixed in 0.8 ml of buffer solution and the DNA was transduced into the cells by electrical pulse with Gene Pulser (Bio-Rad, 400V, 25 µF, electrode width 0.4 cm). After electric pulse, the cells were cultured on feeder cells, which had been cultured on the bottom of 10 cm dish as a monolayer. As feeder cells, fetal fibroblast cells from 12.5 days fetus were inactivated by the treatment with mitomycin C at 0.1 mg/ml for two hrs and lost their mitotic activity. The ES cells were added an antibiotic puromycin at 1.33 µg/ml at 24 hrs, 60 hrs and 96 hrs after start of the culture, continued another 12 hrs, washed and changed to normal medium. By repeating these procedures, puromycin resistant ES clones, which incorporated reporter vector in their chromosome, were selected.

At seven days after start of the culture, about 200 puromycin resistant colonies were transferred 96 well plates and the culture was continued. When cells were sufficiently grown, each plate was divided into three 96 well plates. After the cell were grown furthermore, the first plate was saved at - 85 degree C in a freezer, the second one was used for assay of beta-galactosidase activity and the third one was used for assay of beta-galactosidase activity after introduction of Cre expression vector (pBSCAGCre).

E. coli beta-galactosidase gene in reporter-cells is silent because of upstream presence of puromycin gene and STOP sequence, intervened between two LoxP sequences, and is not translated to protein. Therefore, E. coli beta-galactosidase activity is not detected in reporter-cells without Cre expression. Several clones were selected from cells with beta-galactosidase activity only after introduction of a Cre expression vector, grown and saved in frozen state as reporter-ES cells for gene-trap. Transduction of Cre forced expression vector pBSCAGCre into cells was according to Fugenekit from Rosch.

### Example 4: Transduction of a gene-trap vector into reporter ES cells and selection of candidate cells of gene-trap.

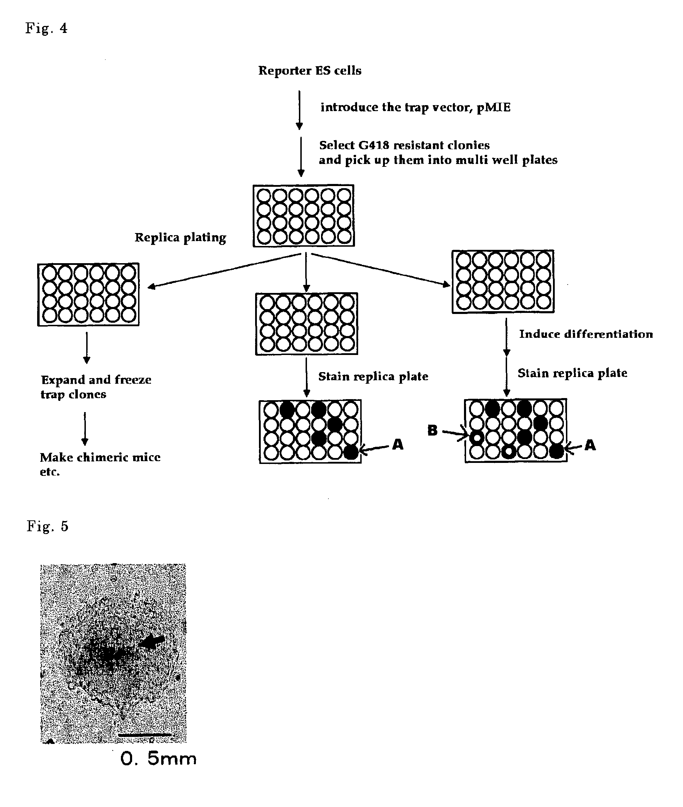

Figure 4 shows a schematic diagram explaining each procedure in this Example. 5 x10⁷ reporter-ES cells and 50 µg of the trap vector pMIE, after linearlized with PmeI and SalI, were mixed and the linearlized vector was transduced into the cells by electrical pulse with Gene Pulser (Bio-Rad, 400V, 25 µF, electrode width 0.4 cm). At 24 hrs after the transduction, an antibiotic G418 was added to the medium at 160 µg titre/ml and the selection of the resistant colonies was started. On 7days after the start of the culture, about 200 colonies of G418 resistant cells were transferred to 96 well plates and the cells were left to grow sufficiently.
At this stage, G418 resistance of cell clones was ascribed to a stable transcription and translation started from Neo gene, which guaranteed trapping an exon of a gene by the trap vector via the second splicing donor added to downstream of Neo gene, or trapping directly poly A addition signal by the trap vector.

Then, each plate was divided into three plates to prepare replicas. They were left to grow and one plate was frozen in a freezer at - 85 degree C for storage (Fig. 4 left), the second plate was used to assay beta-galactosidase activity (Fig. 4 center) and the third plate was used to assay beta-galactosidase activity after differentiation (Fig. 4 right).
After that, *in vitro* induction of differentiation and LacZ staining was performed according to the method reported (Gajovic S. et. al., Experimental Cell Research 242(1), 138-143 (1998)).

The 96 well plate (Fig. 4, center) was used to confirm the presence of LacZ positive cells after fixation by 4 % para-formaldehyde and staining LacZ. Mark A in Fig. 4 center represents LacZ positive.

Then, cells in the another 96 well plate (Fig. 4, right) were grown thoroughly in a feeder layer, briefly trypsinized and cultured in a non-coated plate to form an embryonal body for 4 days in the presence of DMEM medium (5% fetal calf serum) containing 0.001mM retinoic acid. Then, the cells were transferred to a culture dish coated with gelatin and were cultured for 4 days. The embryonal body was adhered to the surface of the culture dish losing its structure and was differentiated to various cell types including neuronal cells. Then, cells were fixed with 4 % para-fromaldehyde, stained for LacZ activity and verified the presence or absence of LacZ positive cells. A part of cells adhered to the culture dish was confirmed to be positive LacZ activity. In Fig. 4 right, marks A and B represent LacZ positive.

The photographs in Fig 5 show LacZ positive cells only after induction of differentiation in the two plates (Fig. 4 center and right). These photographs show that genes, which induce the expression of Cre gene of the trap vector during or after the differentiation of ES cells, were trapped.

### Example 5: Identification of trapped gene by RT-PCR

The procedures were essentially according to the method reported (Frohman M.A. et. al., Proc Natl Acad Sci USA 85(23):8998-9002 (1988)).
Since PGK promoter in the trap vector has constitutive transcriptional activity, there is Neo gene transcripts in antibiotic G418 resistant cells irrespective of differentiated or undifferentiated states. Furthermore, the trapped gene sequence is located between Neo and poly A sequences, due to the characteristics of the present trap vector. Therefore, the trapped gene could be identified and analyzed by PCR amplification between poly A sequence of Neo gene transcripts and Neo gene sequence, after RNA is extracted from a mass cultured trapped clone undifferentiated ES cell.

RNA was extracted from ES cell trap clone using TRIzol kit (Invitrogen). The cDNA was prepared by reverse transcription using dT17 adapter primer (SEQ ID NO: 7) and the isolated RNA as a template. cDNA synthesis was performed using a cDNA synthesis kit (Promega). The first PCR was performed using adapter primer (SEQ ID NO: 8), a primer, which is a part of Neo gene sequence (the first NEO primer, SEQ ID NO: 9), and a part of synthesized cDNA. Then, to increase the efficiency of amplification and the purity, the second PCR was performed using adapter primer (SEQ ID NO: 10) and the second NEO primer comprising sequence located downstream of the first primer.

Based on the above procedures, it was confirmed that identified gene is a gene with a new DNA sequence (data not shown) and that the trap system is functional.

## Claims

1. A method for gene-trap comprising steps of transforming target cells with a reporter vector, transfecting the transformed cells in the previous step with a trap vector, selecting cells without reporter activity from cells obtained in the previous step, exposing the selected cells in the previous step to a given condition and selecting the cells with reporter activity from the cells of the previous step, wherein the reporter vector having a unit composed by connecting, in sequence, promoters functional in the target cells, the first LoxP sequence, drug resistance gene, STOP sequence for transcription termination, the second LoxP sequence and a reporter gene, and the gene-trap vector having a unit composed by connecting, in sequence, splicing acceptor, IRES, Cre added with nuclear localization signal, the first splicing donor, constitutively expressing gene promoter, drug resistance gene and the second splicing donor.

2. The method as in claim 1 further comprising a step of cloning the DNA proximal to the trap vector in the selected cells and determining the nucleotide sequence.

3. The method as in claim 1 or 2, wherein said target cells are ES cells.

4. A set of vectors for gene-trap comprising a reporter vector and a gene-trap vector, wherein said reporter vector comprises a unit composed by connecting, in sequence, promoters functional in the target cells, the first LoxP sequence, drug resistance gene, STOP sequence for transcription termination, the second LoxP sequence and a reporter gene, and said gene-trap vector comprises a unit composed by connecting, in sequence, splicing acceptor, IRES, Cre added with nuclear localization signal, the first splicing donor, constitutively expressing gene promoter, drug resistance gene and the second splicing donor.

5. The cells transformed by the set of vectors as in claim 4 or non-human animal containing said cells.
